# EUROPEAN PATENT SPECIFICATION

(11) **EP 1 695 680 B1**
(45) Date of publication and mention of the grant of the patent: **22.08.2007**
(21) Application number: 05447042.2
(22) Date of filing: 24.02.2005
(51) Int. Cl.: A61F 13/26

(54) **A tampon applicator**
Tamponeinführhilfe
Applicateur de tampon

(43) Date of publication of application: 30.08.2006
(73) Proprietor: Ontex International N.V., 9240 Zele (BE)
(72) Inventor: van Ingelgem, Werner c/o Ontex International N.V., 9240 Zele (BE); De Poorter, Annick c/o Ontex International N.V., 9240 Zele (BE); Smet, Steven c/o Ontex International N.V., 9240 Zele (BE)
(74) Representative: Brants, Johan P.E.

(56) References cited:
- EP-A- 0 355 396

## Description

### FIELD OF THE INVENTION

The present invention relates to tampon applicators and, more particularly, is directed to compact catamenial tampon applicators of the type employing telescoping tubes comprising restraining means for interlocking said tubes.

### BACKGROUND OF THE INVENTION

Most commercially available tampon applicators for introducing catamenial tampons intravaginally consist of a pair of telescoping tubes.

In a compact tampon applicator design the ejector tube is telescoped completely into the outer tube while the tampon is stored in the distal end of the ejector tube. Operatively, the ejector tube is "cocked" by being withdrawn proximally out most of the way from its stored position in the outer tube (leaving behind the tampon) until the distal end of the ejector tube is positioned to engage the proximal end of the stored tampon. Different ways of securing the stored tampon in the distal end of the outer tube to prevent proximal displacement of the tampon while the ejector tube is being withdrawn there from are known in the art.

Applicators of the telescoping tube type typically include a restraining means to prevent disassembly. Interlocking restraining means became more important in preventing disassembly in the compact applicator designs, because the added cocking step needs to be controlled to prevent disassembly. Earlier designs tended to be inadequate because of often being subject to wobble, i.e., lateral movement or swaying of the ejector tube relative to the outer tube, in the fully cocked position. Such wobble reduced the strength of interlocking, thereby increasing the risk of disassembly. The tendency to wobble is also more likely in molded plastic applicators, which because of manufacturing requirements, are made from tubes which are slightly tapered.

EP 0 355 396 B1 disclosed a compact tampon applicator having an improved restraining means for interlocking telescoped tubes without wobble and optionally with audible or sensible seating in the cocked position. The restraining means comprised a pair of two raised rings on the inner surface of the outer tube at its proximal end, the respective facing slopes of which defined an interposed valley. Another raised ring-like structure was included on the outer distal surface of the ejector tube. When the ejector tube was withdrawn from the outer tube in the proximal direction, the ring-like structure on the ejector tube became engaged (i.e., interlocked) within the valley on the outer tube, which prevented the disassembly of the outer and ejector tubes. In interlocked position, all surfaces of the ring-like structure on the ejector tube were closely engaged with the respective facing surfaces of the valley on the outer tube. This restricted lateral movement of the ejector tube and thereby limited the wobble of the ejector tube relative to the outer tube.

The solution provided by EP 0 355 396 B1 to the problem of lateral wobble of the ejector tube relative to the outer tube carries several shortcomings.

First, the wobble was only prevented if all surfaces of the ring-like structure on the ejector tube simultaneously contacted the respective facing surfaces of the valley on the outer tube. However, this required that the dimensions and shapes of the ring-like structure and of the valley had to be perfectly complementary. Such level of precision is not easy to obtain, given the fact that the respective interlocking structures are located on different components of the applicator, i.e., on the outer and ejector tubes, which are manufactured separately an often using different machinery or even at different premises. Hence, production of these components requires a great deal of optimization and meticulous inspection of the production parameters. This difficulty becomes more pronounced because of the very small size of the interlocking structures, whereby any relatively minor deviation in the production process may result in structures which do not anymore contain exactly complementary surfaces and therefore are useless, since they are not able to restrict the wobble.

Second, due to the exact match between the ring-like structure and the valley, the ring-like structure was "snapped" within the valley. Therefore, the user could not sense how much force was needed to release the ring-like structure from the valley, such as when pushing the ejector tube back into the outer tube to dispose the tampon, so that releasing the ring-like structure from the valley was often accompanied with an unpleasant jerk.

Third, in EP 0 355 396 B1 the entire task of preventing the wobble relied on the interlocked rings. However, the interlocked rings could not fully prevent the wobble or lateral swaying of the ejector tube, but rather provided a sort of hinge around which the wobble took place. The close engagement of the interlocked rings could only partially counteract the wobble once this had occurred. Moreover, if the close engagement of the interlocked rings failed, e.g., because of a slight misalignment of the respective facing surfaces, the wobble and subsequent disassembly of the applicator became very likely. To counteract this problem, EP 0 355 396 increased the width of the distal one of the two rings on the outer tube, so that this could engage with the distal portion of the ejector tube. However, increasing the width of the ring also increased the amount of raw material needed to produce the outer tube, which may have a negative impact on the cost of mass production.

Fourth, EP 0 355 396 B1 only provided for close engagement between the outer and ejector tubes when these were in interlocked position. Hence, wobble remained when the ejector tube was only partially withdrawn from the outer tube, but not yet in interlocked position. Although such wobble does not result in disassembly, it tends to complicate the manipulation of the applicator.

To overcome the above shortcomings, the present invention provides a novel and improved restraining means for use in tampon applicators of the telescoping tubes type.

### SUMMARY OF THE INVENTION

The present invention provides a tampon applicator of the type employing telescopic tubes, comprising a novel restraining means for preventing the disassembly of the tubes when said tubes are pulled apart in normal use. The restraining means of the present applicator advantageously reduces lateral wobble between the applicator tubes, both while the tubes are only partially removed from each other, as well as when the restraining means is in interlocked position, i.e., when the structures of the restraining means are mutually interlocked and the applicator is cocked. Limiting of the wobble greatly reduces the likelihood of disassembly.

Accordingly, the present invention provides a tampon applicator comprising an ejector tube, an outer tube dimensioned to fit closely and telescopically over said ejector tube and having a distal discharge end, and a restraining means between said tubes for preventing the disassembly of the applicator when said ejector tube is axially withdrawn from said outer tube in the proximal direction in normal use, characterized in that said restraining means comprises a circumferentially-extending raised means and a set of at least three adjacent circumferentially-extending raised rings.

Hence, while the prior art has only provided for a pair of two raised rings on the inner surface of the outer tube, said pair forming a valley which could accommodate a corresponding ring-like raised means on the outer surface of the ejector tube, the present invention provides for at least one additional ring on the inner surface of the outer tube, which contacts or is in close proximity with the outer surface of the ejector tube. The additional ring provides an extra means of restricting the lateral movement and the resulting wobble of the ejector tube relative to the outer tube. Preferably, the additional ring may be provided close to the proximal end of the outer tube, that is at a location where it can maximally restrict the lateral movement of the ejector tube. Advantageously, the additional ring may be spaced away from the interlocked rings, whereby the applicator comprises at least two relatively distant points of close contact between the surfaces of the ejector tube and the outer tube. These points of contact may work synergistically to inhibit the lateral wobble of the ejector tube.

The provision of the additional ring restricting the lateral movement of the ejector tube removes the requirement for an exact match between the respective facing surfaces of the interlocked structures, i.e., the raised means and the valley, as the wobble is prevented already if only one surface of the raised means closely engages with one corresponding surface of the valley. This improvement greatly simplifies the production process, as it is not anymore absolutely critical that the corresponding interlocking structures have perfectly complementary dimensions and shapes.

Advantageously, the valley may be broader than the raised means and may provide for some freedom of axial movement of the ejector tube in interlocked position. Therefore, the user will be able to sense when the boundaries of such freedom of axial movement are being approached and may adequately increase the force in order to smoothly overcome these boundaries. In particular, this may allow the user to smoothly push the ejector tube from interlocked position back into the outer tube during disposal of the tampon.

The present invention also removes the need for increasing the width of the distal one of the two rings on the outer tube, which may result in savings of raw material and decreased cost of production.

Furthermore the present invention also provides for restricting the wobble not only in interlocked position, but also when the ejector tube is less removed from the outer tube, which simplifies the manipulation of the applicator.

### BRIEF DESCRIPTION OF FIGURES

**Figure 1** illustrates a side view of an embodiment of the tampon applicator of the present invention in cocked position.
**Figure 2** illustrates the restraining means of the tampon applicator of Figure 1.
**Figure 3** illustrates an expanded view of the circled area in Figure 2.

### DETAILED DESCRIPTION OF THE INVENTION

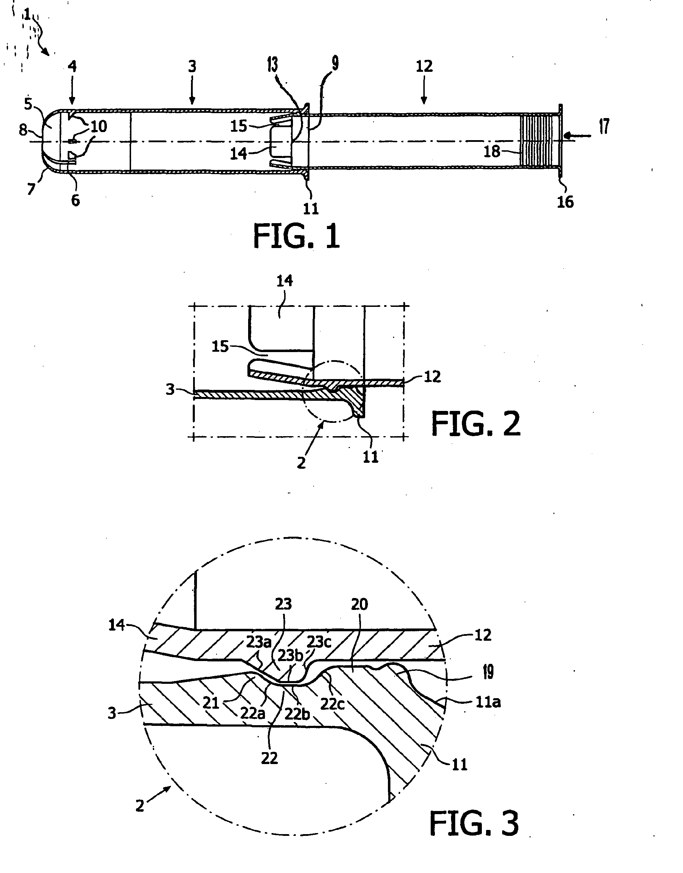

The present invention provides a tampon applicator of the type employing telescopic tubes, comprising a novel restraining means. The novel restraining means may be used with essentially any tampon applicator of the abovementioned type. Figure 1 illustrates a preferred, but non-limiting example of such applicator 1 which comprises an ejector tube 12 adapted to store a tampon therein and an outer tube 3 slideably disposed over the ejector tube 12. The outer tube 3 is adapted to pass the tampon through its distal end 4 during an ejection operation.

The cylindrical outer tube 3 has at its distal end 4 conventional petal sections 5 which are separated from each other by respective slots 6. The petal sections 5 are made relatively flexible and are normally biased in a substantially arcuate closed configuration to form a rounded tip 7 having a central opening 8 at the distal end 4. This rounded shape of the distal end 4 helps facilitate the insertion of the applicator 1 into the vaginal cavity. Such outer tubes 3 are preferably constructed from any suitable smooth plastic material and may be, e.g., molded (e.g., injection molded) or prepared from extruded tubing. The opposite or proximal end 9 of the outer tube 3 is open.

The outer tube 3 of the applicator 1 further includes a plurality, i.e., 2 or more, e.g., 2 to 8, inward projections 10 formed along the inner circumference adjacent to the proximal end of the petal sections 5. For example, one such inward projection 10 may be disposed approximately at the base of each respective petal section 5. The inward projections 10 serve to engage the distal end of a tampon which may be stored in the distal end 13 of an ejector tube 12, thereby preventing axial movement of the tampon in the proximal direction. Therefore, the inward projections 10 are often inclined towards the distal end 4, such as to securely grip a tampon and prevent its movement in the proximal direction (similar to the way a speed nut functions, making a unidirectional jamming engagement). These projections 10 may have various shapes known in the art, e.g., they may be flaps disposed in an inward direction generally perpendicular to the axis of the outer tube 3 and preferably canted towards the distal discharge end 4 thereof. In Figure 1 the projections 10 have a substantially right triangular profile with the hypotenuse (i.e., the longest side) extending at an acute angle from the inner wall of the outer tube 3 towards the central opening 8, one of the catheti (i.e., the shorter sides) facing the distal end 4 of the outer tube 3, and the other cathetus being the contact side with the inner surface of the outer tube 3. The projections 10 may be relatively thin with thickness ranging, e.g., from 0.2mm to 5mm.

The outer tube 3 of the present applicator 1 may further comprise an outwardly directed circumferential flange 11 at its proximal end 9. The proximal slope 11a of the flange may serve as a lead-in slope for facilitating the initial assembly of the ejector 12 and outer 3 tubes.

The outer proximal surface of the outer tube 3 may optionally be provided with a ridged or knurled surface, which provides for a firmer grip when this portion of the outer tube 3 is held between fingers.

The outer tube 3 of the present invention further comprises structures of the novel restraining means 2, which are detailed elsewhere in this specification.

The tampon applicator 1 further comprises an inner or ejector tube 12. This serves as a hollow plunger for ejecting a tampon positioned within the distal end 4 of the outer tube 3. Ejector tube 12 may be constructed from any suitable smooth plastic material and may be, e.g., molded (e.g., injection molded) or prepared from extruded tubing. The ejector tube 12 is dimensioned to easily slide within the outer tube 3, with minimal clearance in between. The ejector tube 12 is also preferably slightly longer that the outer tube 3 to assure complete ejection and proper depth of placement of the tampon. Being hollow, the ejector tube also permits proper placement of a withdrawal string usually attached to the proximal end of the tampon. The distal end 13 of the ejector tube 12 is typically formed with a plurality, i.e., 2 or more, e.g., 2 to 8, of fingers 14 which are separated from each other by slots or openings 15, through which the inward projections 10 of the outer tube 3 extend to engage the tampon disposed therein. In the same manner as with petal sections 5 of the outer tube 3, the fingers 14 are made so as to be slightly biased towards a closed configuration, as shown in Figures 1 and 2. Such fingers 14 tend to be considerably shorter than the petal sections 5 of the outer tube 3, e.g., to give a stronger structure. The ejector tube 12 may be functional also without such fingers 14 if a tampon is sufficiently expansive to fit closely within the outer tube 3 to ensure positive engagement by an unmodified distal end 13 of the ejector tube 12 against the proximal end of the tampon during the ejection step.

The ejector tube 12 also includes an outwardly directed circumferential retention flange 16 at its proximal end 17. The flange 16 controls the extent of insertion of the ejector tube 12 into the outer tube 3 of the applicator 1. The flange 16 of the ejector tube 12 will abut the retention flange 11 of the outer tube 3 when the ejector tube 12 is fully inserted into the outer tube 3.

The inner proximal portion of the ejector tube 12 may optionally be provided with a ridged or knurled surface 18, which provides for a firmer grip when a finger is inserted into the ejector tube.

The ejector tube 12 of the present invention further comprises structures of the novel restraining means 2, which are detailed elsewhere in this specification.

When a tampon applicator 1 is prepared for use, the ejector tube 12 is disposed within the outer tube 3 and over a stored tampon. Accordingly, in an aspect, the present invention provides an assembly comprising the applicator 1 of the present invention and a tampon.

The distal end 13 of the ejector tube 12 may abut the inward projections 10. The flexible fingers 14 of the ejector tube 12 may be biased flexibly outward by the carried tampon. This permits the fingers, and the remainder of the ejector tube 12, to pass over the tampon that is secured in the outer tube 3 by the inward projections 10 during partial withdrawal of the ejector tube 12. Then, because of the stiff flexibility of the fingers 14, when the ejector tube 12 is sufficiently withdrawn, the fingers will flex inwardly to a lesser diameter than that of the tampon. Consequently, when the ejector tube 12 is axially moved in the distal direction, the tampon will be engaged at its proximal end by the fingers 14 and urged out the distal discharge end 4 of the outer tube 3.

Accordingly, the present invention further provides for use of the applicator 1 of the present invention to discharge a tampon. Also, the present invention provides for use of the assembly of the applicator 1 and a tampon to discharge said tampon. The tampon is in particular a catamenial tampon and may be discharged within a body cavity, in particular vaginal cavity. Thereby, the tampon may be placed within said cavity.

The present applicator 1 may be used in conjunction with any tampons, in particular catamenial tampons, known in the art. Tampons for use with the present applicator 1 may comprise known modifications which may improve the working of the telescoping tubes type of applicators. In a non-limiting example, the base of the tampon may be uniquely formed with a concave configuration leaving a more dense center and a relatively softer peripheral ridge. The softer ridge may aid the proximal end of the tampon in catching on the fingers 14, thus serving as an unidirectional lock to prevent the tampon from re-entering the ejector tube 12 during expulsion. In another example, the base of the tampon need not have a concave configuration. In another example, the tampon may be a two-diameter tampon. In an example, the tampon may comprise a slightly enlarged head.

In a particularly useful example, the present applicator 1 may be used in conjunction with (so-called) digital tampons. Accordingly, in an embodiment, the present invention provides for use of the applicator 1 of the present invention to discharge a digital tampon. In an embodiment, the present invention provides an assembly comprising the applicator 1 of the present invention and a digital tampon. In an embodiment, the present invention provides for use of the assembly of the applicator 1 and a digital tampon to discharge said digital tampon. The tampon may be discharged within a body cavity, in particular vaginal cavity. Thereby, the tampon may be placed within said cavity.

Digital tampons are typically inserted into the vaginal cavity without the assistance of an applicator and have useful properties which make them suitable for this way of insertion. For example, digital tampons may be manufactured in small size in order to make their insertion more comfortable. Because of their small size, they tend to have high absorption and expansion capacity.

Generally, tampons are manufactured from absorbent materials, such as rayon, cotton, or their mixtures. For digital tampons, the volume of absorbent material necessary to provide sufficient absorption capacity must be highly compressed to form cylindrical tampons of small size. The compression is adequate to hold the tampon in cylindrical shape until insertion is completed and the tampons may therefore have relatively high initial density.

A digital tampon comprises an insertion end (i.e., distal end), a recovery end (i.e., proximal end) with a withdrawal string, and a central section extending there between. Typically, a digital tampon has a compressed, generally cylindrical, solid fibrous core, from which relatively uncompressed longitudinal ribs extend radially outward. Such ribs may assume various shapes e.g., straight, sinusoidal, spirally or helically shaped in the axial direction between the insertion end and the withdrawal end. Exemplary tampons of this type are disclosed in, e.g., WO 02/078586, EP 0 422 660 and EP 0 639 363.

In a particularly useful example, the digital tampon may contain one or more ribs that in transverse cross-section may have a median at least partially diverging from the radius, essentially as described in EP 04447289.2. Such tampons provide high absorption and expansion capacity and are comfortable in use. Accordingly, in an embodiment, the present invention provides for use of the applicator 1 of the present invention to discharge a digital tampon, wherein said tampon comprises at least one rib that in transverse cross-section has a median at least partially diverging from the radius. In an embodiment, the present invention provides an assembly comprising the applicator 1 of the present invention and a digital tampon, wherein said tampon comprises at least one rib that in transverse cross-section has a median at least partially diverging from the radius. In an embodiment, the present invention provides for use of the assembly of the applicator 1 and a digital tampon to discharge said digital tampon, wherein said tampon comprises at least one rib that in transverse cross-section has a median at least partially diverging from the radius. The tampon may be discharged within a body cavity, in particular vaginal cavity. Thereby, the tampon may be placed within said cavity.

The number of the ribs can vary, e.g., depending on the diameter of the tampon and/or the type of absorption material, there may be between 4 and 12 ribs, and often about 8. There may be an even or odd number of such ribs. The ribs increase the surface of the tampon that is available for absorption of fluids, thus increasing the absorption capacity of the tampon. The body fluid also has to cover longer distances when such longitudinal ribs are provided, thus increasing the dwell time of the liquid in the longitudinal grooves extending along the surface of the tampon. Hence, the contact and absorption of the body fluid by the tampon is increased and the likelihood of leakage is decreased. The risk of leakage may further be reduced if the fiber core of the tampon is pressed more strongly in the central area than in the area of the recovery end of the tampon.

Preferably, before use, the ribs fit closely together near the circumferential surface, providing an essentially cylindrical, smooth and soft surface. This makes insertion of the tampon more comfortable. The feeling of comfort upon insertion may further be enhanced if the tampon is at least partially surrounded by a liquid-permeable smooth sheathing.

Digital tampons may be formed, e.g., as follows: rolling up a length of a continuous fibrous web to form a generally cylindrical tampon blank with a circumferential surface; simultaneous radial pressing of narrow, strip-shaped sections of the circumferential surface of the tampon blank arranged in a spaced manner to form a number of longitudinal grooves which are separated from one another by relatively uncompressed longitudinal ribs which extend radially outward from a relatively compressed core, the core being compressed to a smaller extent in the area of the recovery end of the tampon than in its remaining area; and pressing of outer ends of the longitudinal ribs to form a soft, smooth circumferential surface, while the relatively uncompressed fibrous structure of the ribs is preserved. A round dome can be provided at the insertion end of the tampon and an optional finger recess at recovery end. A liquid-permeable sheathing may be fixed on the fibrous web. In a particularly useful example, the tampon may be produced using a process and press as described in EP 04447289.2.

Reduced size, higher compression and higher absorption capacity of digital tampons are properties which are likely to be welcomed by most users. However, many users may have reservations, e.g., hygienic or personal, towards inserting such tampons directly by fingers. Hence, providing applicators for this kind of tampons may stimulate such users to employ these tampons and thereby benefit from some of their superior properties, e.g., smaller size and high absorption. In addition, the smaller size of the digital tampons may also allow reduction in the size of the applicator, which may lead to reduction in raw material, which is both economically and environmentally beneficial.

In an aspect, the present invention provides a tampon applicator 1 having a novel restraining means 2 for preventing the disassembly of the ejector tube 12 from the outer tube 3 when the ejector tube 12 is axially withdrawn from the outer tube 3 in the proximal direction in normal use. The restraining means 2 of the present applicator 1 advantageously reduces wobble between the ejector 12 and outer tubes 3, in particular when the restraining means 2 is in interlocked position, i.e., when the structures of the restraining means 2 are mutually interlocked and the applicator is cocked. Reducing the lateral wobble decreases the likelihood of disassembly of the outer 3 and ejector 12 tubes. A preferred, but non-limiting, embodiment of the restraining means 2 is illustrated in Figures 2 and 3.

Accordingly, the present invention provides a tampon applicator comprising:
- an ejector tube 12;
- an outer tube 3 dimensioned to fit closely and telescopically over said ejector tube 12 and having a distal discharge end 4;
- a restraining means 2 between said tubes 12,3 for preventing the disassembly of said ejector tube 12 from said outer tube 3 in the proximal direction;
characterized in that said restraining means 2 comprises:
- a circumferentially-extending raised means 23; and
- a set of at least three adjacent circumferentially-extending raised rings 19,20,21, respectively being a supporting ring 19, a stopping ring 20, and a positioning ring 21.

In an embodiment, the set of rings 19,20,21 is provided on the inner proximal surface of the outer tube 3 and the raised means 23 is provided on the outer distal surface of the ejector tube 12, as shown in Figures 2 and 3.

In a further embodiment, when the rings 19,20,21 are provided on the inner proximal surface of the outer tube 3, the supporting ring 19 is the closest one of the rings 19,20,21 to the proximal end 9 of the outer tube 3. The ring 21 closest to the distal end 4 of the outer tube 3 is the positioning ring 21, and the ring 20 interposed between the positioning ring 21 and the supporting ring 19 is the stopping ring 20.

In the present applicator, the supporting ring 19 restricts the lateral movement of the ejector tube 12. Therefore, placing of the supporting ring 19 to be the closest one of the three rings 19,20,21 to the proximal end 9 of the outer tube 3 allows it to restrict the lateral movement of the ejector tube 12 at a point where this is likely to arise due to manipulation of the ejector tube 12.

In an embodiment, when the rings 19,20,21 are provided on the inner proximal surface of the outer tube 3, the supporting ring 19 is provided close to the proximal end 9 of the outer tube 3. In this position, the supporting ring 19 is able to maximally restrict the lateral movement of the ejector tube 12 at a point where this is likely to arise due to manipulation of the ejector tube 12. Moreover, positioning of the supporting ring 19 close to the proximal end 9 of the outer tube 3 will maximally increase the axial distance of the supporting ring 19 from a valley 22 interposed between the positioning ring 21 and the stopping ring 20. Typically, in the present invention, the surfaces of the outer tube 3 and the ejector tube 12 will be in closest proximity or in contact at the valley 22 and at the supporting ring 19. Increasing the axial distance between these two points will therefore promote their synergistic effect against the lateral movement of the ejector tube 12.

In an embodiment, when the rings 19,20,21 are provided on the inner proximal surface of the outer tube 3, the minimum diameter of the supporting ring 19 is equal to or smaller than the minimum diameter of the stopping ring 20. The term "minimum diameter of a ring" refers to the smallest diameter defined by the ring 19,20,21. Since the rings 19,20,21 protrude from the inner surface of the outer tube 3 into the inner space enclosed by said tube 3, the minimum diameter of each ring 19,20,21 is defined by that portion of said ring where the ring 19,20,21 has maximum height relative to the inner surface of the tube 3. In other words, a ring 19,20,21 has its minimum diameter there, where it maximally protrudes into the inner space enclosed by the outer tube 3. Therefore, when the minimum diameter of one ring 19,20,21 is said to be smaller than the minimum diameter of another ring 19,20,21, then the former ring protrudes more into the inner space enclosed by the outer tube 3 than the latter ring. Hence, in the present embodiment, the supporting ring 19 has equal height or is higher than the stopping ring 20 relative to the inner surface of the outer tube 3, i.e., the supporting ring 19 protrudes to an equal or greater extent into the inner space enclosed by the outer tube 3 than the stopping ring 20 does. Hence, the supporting ring 19 will be equally distanced from or closer to the outer surface of the ejector tube 12 than the stopping ring 20, and thus may be suited to restrict the lateral movement of the tube 12.

In another embodiment, when the rings 19,20,21 are provided on the inner proximal surface of the outer tube 3, the minimum diameter of the supporting ring 19 is equal to or smaller than the minimum diameter of the positioning ring 21. Hence, the supporting ring 19 has equal height or is higher than the positioning ring 21 relative to the inner surface of the outer tube 3, i.e., the supporting ring 19 protrudes to an equal or greater extent into the inner space enclosed by the outer tube 3 than the positioning ring 21 does. Hence, the supporting ring 19 will be equally distanced from or closer to the outer surface of the ejector tube 12 than the positioning ring 21, and thus may be suited to restrict the lateral movement of the tube 12.

In an embodiment, when the rings 19,20,21 are provided on the inner proximal surface of the outer tube 3, the minimum diameter of the supporting ring 19 is equal to or smaller than the minimum diameter of each of the stopping 20 and positioning 21 rings. Hence, the supporting ring 19 has equal height or is higher than each of the stopping 20 or positioning 21 rings relative to the inner surface of the outer tube 3, i.e., the supporting ring 19 protrudes to an equal or greater extent into the inner space enclosed by the outer tube 3 than each of the stopping 20 and positioning 21 rings. Hence, the supporting ring 19 will be equally distanced from or closer to the outer surface of the ejector tube 12 than the stopping ring 20 and the positioning ring 21, and thus may be suited to restrict the lateral movement of the tube 12.

In an embodiment, when the rings 19,20,21 are provided on the inner proximal surface of the outer tube 3, the minimum diameter of the supporting ring 19 is smaller than the minimum diameter of each of the stopping 20 and positioning rings 21. Hence, the supporting ring 19 is higher than each of the stopping 20 and positioning 21 rings relative to the inner surface of the outer tube 3, i.e., it protrudes to a greater extent into the inner space enclosed by the outer tube 3 than each of the stopping 20 and positioning rings 21. In this situation, the supporting ring 19 will protrude closer towards the outer surface of the ejector tube 12 than any of the other two rings 20,21. Hence, the supporting ring 19 may be most suited to restrict the lateral movement of the tube 12.

In an embodiment, when the rings 19,20,21 are provided on the inner proximal surface of the outer tube 3, the minimum diameter of the supporting ring 19 is about the same as the outer diameter of the ejector tube 12. The term "about the same" is intended to mean that the diameters may be identical or that one diameter may differ from the other diameter by 0-10% of the value of the first diameter. Typically, if said diameters are not identical, the minimum diameter of the supporting ring 19 will be slightly (e.g., 0-10%) larger than the outer diameter of the ejector tube 12. Hence, the supporting ring 19 may preferably be in a continuous touching relationship with the outer surface of the ejector tube 12, or may be in very close proximity with the outer surface of the ejector tube 12. Such arrangement may advantageously limit the freedom of lateral movement of the ejector tube 12 when this is inserted within the outer tube 3. This may reduce the lateral wobble of the ejector tube 12 relative to the outer tube 3.

In an embodiment, the supporting ring 19 may be formed by a plurality of circumferentially aligned protrusions. Hence, the supporting ring 19 may not extend continuously along the entire circumference of the respective tube, but may be formed by a plurality, i.e., 2 or more, e.g., 2 to 10, of protrusions, each extending over a portion of the circumference defined by a radial angle. The radial angle may range, e.g., from 1° to 360°, wherein 360° degree corresponds to a continuous ring. This alteration may save raw material in production.

In an embodiment, the stopping ring 20 may be formed by a plurality of circumferentially aligned protrusions. Hence, the stopping ring 20 may not extend continuously along the entire circumference of the respective tube, but may be formed by a plurality, i.e., 2 or more, e.g., 2 to 10, of protrusions, each extending over a portion of the circumference defined by a radial angle. The radial angle may range, e.g., from 1° to 360°, wherein 360° degree corresponds to a continuous ring. This alteration may save raw material in production.

In an embodiment, the positioning ring 21 may be formed by a plurality of circumferentially aligned protrusions. Hence, the positioning ring 21 may not extend continuously along the entire circumference of the respective tube, but may be formed by a plurality, i.e., 2 or more, e.g., 2 to 10, of protrusions, each extending over a portion of the circumference defined by a radial angle. The radial angle may range, e.g., from 1° to 360°, wherein 360° degree corresponds to a continuous ring. This alteration may save raw material in production.

In an embodiment, at least one of the stopping 20 and positioning 21 rings may be formed by such plurality of circumferentially aligned protrusions, each extending over a portion of the circumference defined by a radial angle, ranging, e.g., from 1° to 360°.

In further embodiments, any one, any two, or all three of the rings 19,20,21 may be formed by such plurality of circumferentially aligned protrusions, each extending over a portion of the circumference defined by a radial angle, ranging, e.g., from 1° to 360°.

In an embodiment, the respective facing slopes 22c,22a of the stopping ring 20 and the positioning ring 21 at least partially define a valley 22 in between. The term "at least partially define" is intended to mean that the valley is defined by at least a portion of each respective facing slope 22c,22a. When the rings 19,20,21 are provided on the inner proximal surface of the outer tube 3, the valley 22 is defined by at least portions of the proximal slope 22a of the positioning ring 21 and the distal slope 22c of the stopping ring 20.

In the step of cocking the applicator 1, the ejector tube 12 may be axially withdrawn in the proximal direction until the raised means 23 has passed the positioning ring 21 and became engaged (i.e., interlocked) within the valley 22 so that further axial movement of the ejector tube 12 in the proximal direction is prevented. Accordingly, in an embodiment, the valley 22 is able to engage the raised means 23.

In a further embodiment, the valley 22 is able to engage the raised means 23 with a degree of freedom, i.e., when the raised means 23 is engaged within the valley 22, the ejector tube 12 retains a certain, usually small, freedom of axial movement. This means that the ejector tube 12 can be axially moved in the proximal and distal directions to a certain extent. The force needed to obtain such movement may usually be similar to the force needed to achieve the ordinary telescopic sliding of the tubes 3,12.

Typically, a valley 22 which can engage a raised means 23 with a degree of freedom will be broader than the raised means 23. Hence, unlike in EP 0 355 396 B1, in the present invention the dimensions and shapes of the valley 22 and the raised means 23 need not be exactly complementary. Rather, the valley 22 may be broader than the raised means 23 and the shape of the valley 22 need not exactly match that of the raised means 23. This provides advantages in production, as it is much easier to produce outer tubes 3 comprising valleys 22 which may be broader than the corresponding raised means 23 on the ejector tubes 12 and may have relatively relaxed shapes, than it is to produce outer tubes comprising valleys with dimensions and shapes exactly complementary to the corresponding raised means on the ejector tubes, as required in EP 0 355 396 B1. In the present invention, exact match is not crucial, because the supporting ring 19 provides an additional element counteracting the lateral wobble of the ejector tube 12.

Moving the ejector tube 12 beyond the boundaries of the freedom of axial movement when the raised means 23 is engaged within the valley 22 will typically require considerably more force in order to overcome the positioning ring 21 or the stopping ring 20, which provide barriers to axial movement in the distal and proximal direction, respectively. However, because the valley 22 may be broader than the raised means 23 and may provide for some freedom of axial movement of the interlocked ejector tube 12, the user will be more able to sense when the boundaries of the freedom of axial movement are being approached and may adequately increase the force in order to smoothly overcome these boundaries. In particular, this may allow the user to smoothly overcome the positioning ring 21 when the ejector tube 12 is pushed back into the outer tube 3 during disposal of the tampon. In contrast, in EP 0 355 396 B1 the raised means was "snapped" in the valley and the user could not sense how much force was needed to release it, so that the raised means was often released with an unpleasant jerk.

When the raised means 23 is engaged in the valley 22, the surfaces 23a,23b,23c of the raised means 23 may at least partially contact the surfaces 22a,22b,22c of the valley 22. Advantageously, these mutual contacts and/or close proximity of such opposing surfaces may limit the freedom of lateral movement of the ejector tube 12 when the restraining means 2 is in interlocked position. This may reduce the lateral wobble of the ejector tube 12 relative to the outer tube 3 when the restraining means 2 is in interlocked position, i.e., when the applicator 1 is in cocked position. Such mutual contacts between the surfaces 23a,23b,23c,22a,22b,22c, of the raised means 23 and of the valley 22 may become more extensive, which may advantageously provide for greater lateral stabilization of the ejector tube 12 in the interlocked position, when at least some of the surfaces 23a,23b,23c,22a,22b,22c involved in such contacts have a corresponding shape and/or size. Opposing surfaces having corresponding shape and/or size may make better contacts than opposing surfaces having dissimilar shapes and/or sizes. For example, if the valley 22 comprises a substantially flat bottom, the raised means 23 may comprise a parallel plateau of a comparable size, such that maximal contact would be obtained. In another example, the raised means 23 comprises at least one slope 23a,23c that is similar in shape and/or size to a slope 22a,22c of the valley 22. Such slopes may align and make contacts and may become aligned when the raised means 23 is engaged within the valley 22. Slopes of corresponding shape may, e.g., be inclined under similar angles with respect to the longitudinal axis. These angles may be identical or may differ by a small amount, e.g., by up to 15°.

Accordingly, in an embodiment the raised means 23 comprises at least one surface 23a,23b,23c corresponding in its shape and/or size to a surface 22a,22b,22c of said valley 22.

In another embodiment, when the rings 19,20,21 are provided on the inner proximal surface of the outer tube 3 and the raised means 23 is provided on the outer distal surface of the ejector tube 12, the raised means 23 comprises a distal slope 23a corresponding in its shape and/or size to distal slope 22a of the valley 22. The distal slope 22a of the valley 22 is defined by the proximal slope of the positioning ring 21.

This arrangement is particularly advantageous because the axial distance between the sites of contact and/or close (or even closest) proximity between the opposing surfaces of the outer tube 3 and ejector tube 12 may be maximized when the restraining means 2 is in the interlocked position. One site of contact and/or close (or closest) proximity is between the distal slope 23a of the raised means 23 and the distal slope 22a of the valley 22. Another site of contact and/or close (or closest) proximity is between the supporting ring 19 and the outer surface of the ejector tube 12. Each of these sites of contact or close proximity may on its own limit the freedom of lateral movement of the ejector tube 12 relative to the outer tube 3, when the restraining means 2 is in interlocked position.

Advantageously, the freedom of lateral movement of the ejector tube 12 and the lateral wobble may further be limited by these two sites working in synergy. Such synergistic working may be more pronounced when these sites are farther away from each other. One means to achieve increased axial distance between the two sites is when the interaction between the raised means 23 and the valley 22 takes place between the most distal surfaces 22a,23a of these elements 22,23.

When the raised means 23 is provided on the outer distal surface of the ejector tube 12, the maximum diameter of the raised means 23, i.e., the diameter at that portion where the raised means 23 maximally protrudes from said surface of the ejector tube 12, is typically greater than the minimum diameter of each of the rings 19,20,21 provided on the inner proximal surface of the outer tube 3. Hence, the raised means 23 at least partially overlaps with the rings 19,20,21 in radial direction. The amount of the overlap is preferably such that the positioning ring 21 (which is the most distal of the three rings 19,20,21 and has the greatest minimum diameter) allows the raised means 23 to pass beyond the positioning ring 21 in the proximal direction in normal use, such that the raised means 23 becomes engaged in the valley 22, and the stopping ring 20 (which usually is proximal to the positioning ring 21 and has a smaller minimum diameter than the positioning ring 21) effectively obstructs the raised means 23 from passing beyond the stopping ring 20 in the proximal direction in normal use. Hence, the stopping ring 20 prevents the disassembly of the applicator 1 in normal use. The supporting ring 19 (which usually is proximal to the stopping ring 21 and has a smaller minimum diameter than the stopping ring 21) restricts the lateral movement of the ejector tube 12 in this position.

In an embodiment, the raised means 23 is formed by a plurality, i.e. 2 or more, e.g., 2 to 12, of circumferentially aligned protrusions. Each such protrusion extends over a portion of the circumference defined by a radial angle. The radial angle may range, e.g., from 1° to 360°, wherein 360° degree corresponds to a continuous ring. The protrusions may preferably be equally spaced along the circumference of the ejector tube 12. The protrusions of the present invention may be considerably shorter (or thinner) in the circumferential direction than the four "ribs" 234 of Figure 13 of EP 0 355 396, which span almost the entire circumference of the ejector tube. Using such shorter protrusions, e.g., between 0.1 mm and 4mm in the circumferential direction, may lead to raw material savings.

When both the raised means 23 and at least one of the rings 19,20,21, and particularly any one or both of the positioning 21 and stopping 20 rings, are formed by a plurality of circumferentially aligned protrusions, the radial angles defining the protrusions of the raised means 23 will preferably be greater than the radial angles defining the circumferential gaps between the protrusions of the respective ring 19, 20, 21. Hence, the length (or thickness) of the protrusions of the raised means 23 in the circumferential direction will be greater than the circumferential length of gaps between the protrusions of the respective ring 19,20,21. This will ensure that the protrusions of the raised means 23 cannot freely pass through the gaps between the protrusions of the respective ring 19,20,21, in particular the positioning 21 and/or stopping 20 ring, when the ejector tube 12 is withdrawn in the proximal direction. Rather, the protrusions of the raised means 23 should engage with the protrusions forming the respective ring 19,20,21, in particular the positioning 21 and/or stopping 20 ring, in a manner functionally equivalent to the situation where these structures would be full rings.

The working and further advantages of the present invention are further explained with reference to a preferred embodiment, the restraining means 2 of which is illustrated in interlocked position in Figure 3. The particular features of this embodiment are only exemplary and do not limit the scope of protection in any way.

The rings 19,20,21 are provided on the inner proximal surface of the outer tube 3 and the raised means 23 is provided on the outer distal surface of the ejector tube 12. The supporting ring 19 is located at the very proximal end 9 of the outer tube 3. The proximal slope of the supporting ring 19 continues directly into a lead-in slope 11 a which aids the insertion of the ejector tube 12 into the outer tube 3 upon assembly of the applicator 1.

Distally to the supporting ring 19 is located in this order the stopping ring 20, the valley 22 and the positioning ring 21. The minimum diameter of the supporting ring 19 is smaller than that of the stopping ring 20 and the minimum diameter of the stopping ring 20 is smaller than that of the positioning ring 21. The supporting ring 19 is in very close proximity or in contact with the outer surface of the ejector tube 12, thereby limiting the freedom of lateral movement of the ejector tube 12.

The raised means 23 is engaged within the valley and the distal slope 23a of the raised means has a shape corresponding to the distal slope 22a of the valley, and these two slopes are in very close proximity or mutual contact.

The stopping ring 20 is prolonged in the longitudinal cross-section and comprises an extended plateau. The extended shape of the stopping ring 20 provides for suitable spatial separation between the valley 22 and the supporting ring 19, and thereby between the site of contact between the distal slope 23a of the raised means 23 and the distal slope 22a of the valley 22 and the site of contact between the supporting ring 19 and the outer surface of the ejector tube 12. The spatial separation of these two sites of contact and/or close (or closest) proximity provides for synergistic inhibition of the lateral movements of the ejector tube 12 when the restraining means 2 is in interlocked position.

The raised means 23 may comprise a distal slope 23a and a proximal slope 23c. Typically, the angle between the distal slope 23a and the longitudinal axis of the tube may be smaller (e.g., ranging from 1 ° to 40°) than that between the proximal slope 23c and the axis of the tube (e.g., up to 90°). Therefore, the distal slope 23a may emerge more gradually, while the proximal slope 23c may rise more steeply. This would ensure that dislocating the raised means 23 from the valley 22 when the ejector tube 12 is pressed from the interlocked position back into the outer tube 3 will require considerably less force than dismembering the applicator 1 by pulling the ejector tube 12 entirely out of the outer tube 3.

As already referred to, the present arrangement of elements forming the restraining means 2 counteracts wobble inherent with this kind of applicators when they are in the cocked position in a manner different from prior art. For example, in EP 0 355 396 the wobble was minimized due to close fitting between the "ribs" 234 on the ejector tube and both slopes of the "valley" 272 on the outer tube. In the present invention, contact and/or close proximity between the outer surface of the ejector tube 12 and the inner surface of the outer tube 3 is provided at two sites, namely between the raised means 23 and the valley 22, and between the supporting ring 19 and the opposing surface of the ejector tube 12. Close association of surfaces at these two positions removes a great deal of lateral movement at these points. Moreover, the axial distance between these two contact points may be increased by provision of an (extended) stopping ring 20, which further synergistically stabilizes the ejector tube 12 against wobble.

Typically, the distal slope of the positioning ring 21 may rise less steeply (i.e., forms a smaller angle with the tube axis, e.g., ranging from 5° to 40°) than its proximal slope 22a. This ensures that pulling the ejector tube 12 axially in the proximal direction into the interlocked position will require comparably less force than the initial push required to dislocate the ejector tube 12 from the interlocked position when it is being pushed back into the outer tube 3.

While the above description focused on embodiments wherein the set of rings 19,20,21 is provided on the inner proximal surface of the outer tube 3 and the raised means 23 is provided on the distal outer surface of the ejector tube 12, it is also possible to exchange these two elements between the tubes, so that the set of rings 19,20,21 is formed on the outer distal surface of the ejector tube 12 and the raised means 23 is formed on the inner proximal surface of the outer tube 3. Here, the supporting 19 ring is the outermost of the three rings 19,20,21, i.e., it is closest to the distal end 13 of the ejector tube 12, and the positioning ring 21 is the innermost of the three rings, i.e., it is closest to the proximal end 17 of the ejector tube 12, while the stopping ring 20 is interposed in between the supporting 19 and positioning 21 rings. Other considerations for this alternative are analogous to the above described alternative and will be immediately obvious to a skilled person.

When the present applicator 1 is used and the ejector tube 12 is pulled into the interlocked position, the tube must first pass beyond the positioning ring 21. The friction between the raised means 23 and the positioning ring 21 during this step gives an initial "warning" to the user that the interlocked position is to be achieved soon, and "snapping" of the raised means 23 into the valley 22 may produce a sensible (e.g., audible and/or tactile) stimulus, informing the user that the cocking step has been completed.

The restraining means 2 of the present invention may be useful, for example, in tampon applicators having stiffness of 68.9 to 620 MPa (10,000 to 90,000 psi), and especially the more preferred range of 68.9 to 276 Mpa (10,000 to 40,000 psi) (typical of the soft resins like low density polyethylene or linear low density polypropylene, which latter ranges from 103.4 to 551.6 MPa (15,000 to 80,000 psi)).

For example, the present restraining means may have a disassembly force in the range of 1000 gms to 1600 gms, measured as a straight pull.

## Claims

1. A tampon applicator comprising:
- an ejector tube (12);
- an outer tube (3) dimensioned to fit closely and telescopically over said ejector tube (12) and having a distal discharge end (4);
- a restraining means (2) between said tubes (12; 3) for preventing the disassembly of said ejector tube (12) from said outer tube (3) in the proximal direction;
**characterized in that** said restraining means (2) comprises:
- a circumferentially-extending raised means (23); and
- a set of at least three adjacent circumferentially-extending raised rings (19; 20; 21), respectively being a supporting ring (19), a stopping ring (20), and a positioning ring (21).

2. An applicator according to claim 1, wherein said set of rings (19; 20; 21) is provided on inner proximal surface of the outer tube (3) and said raised means (23) is provided on outer distal surface of the ejector tube (12).

3. An applicator according to claim 2, wherein the supporting ring (19) is the closest ring of said set (19; 20; 21) to proximal end (9) of the outer tube (3).

4. An applicator according to claim 3, wherein the supporting ring (19) is provided close to the proximal end (9) of the outer tube (3).

5. An applicator according to any of claims 2 to 4, wherein the minimum diameter of the supporting ring (19) is equal to or smaller than the minimum diameter of the stopping ring (20).

6. An applicator according to any of claims 2 to 5, wherein the minimum diameter of the supporting ring (19) is equal to or smaller than the minimum diameter of the positioning ring (21).

7. An applicator according to any of claims 2 to 6, wherein the minimum diameter of the supporting ring (19) is about the same as the outer diameter of the ejector tube (12).

8. An applicator according to any of claims 2 to 7, wherein the supporting ring (19) is formed by a plurality of circumferentially aligned protrusions.

9. An applicator according to any of claims 2 to 8, wherein at least one of the stopping (20) and positioning (21) rings is formed by a plurality of circumferentially aligned protrusions.

10. An applicator according to any of claims 2 to 9, wherein respective facing slopes of the stopping ring (20) and the positioning ring (21) at least partially define a valley (22) in between.

## Patentansprüche

1. Tamponapplikator mit:
- einer Ausstoßröhre (12);
- einer äußeren Röhre (3), die dafür dimensioniert ist, eng und teleskopartig auf der Ausstoßröhre (12) zu sitzen, und die ein distales Austrittsende (4) aufweist;
- einer Zurückhalteereinrichtung (2) zwischen den Röhren (12; 3) zur Verhinderung der Trennung der Ausstoßröhre (12) von der äußeren Röhre (3) in der proximalen Richtung;
**dadurch gekennzeichnet, daß** die Zurückhalteeinrichtung (2) aufweist:
- eine sich entlang des Umfangs erstreckende erhöhte Einrichtung (23); und
- einen Satz von mindestens drei benachbarten, sich entlang des Umfangs erstreckenden erhöhten Ringen (19; 20; 21), bei denen es sich um einen Stützring (19), einen Anschlagring (20) bzw. einen Positionierungsring (21) handelt.

2. Applikator nach Anspruch 1, wobei der Satz von Ringen (19; 20; 21) auf einer proximalen Innenfläche der äußeren Röhre (3) vorgesehen ist und die erhöhte Einrichtung (23) auf einer distalen Außenfläche der Ausstoßröhre (12) vorgesehen ist.

3. Applikator nach Anspruch 2, wobei der Stützring (19) derjenige Ring des Satzes (19; 20; 21) ist, der dem proximalen Ende (9) der äußeren Röhre (3) am nächsten ist.

4. Applikator nach Anspruch 3, wobei der Stützring (19) nahe dem proximalen Ende (9) der äußeren Röhre (3) vorgesehen ist.

5. Applikator nach einem der Ansprüche 2 bis 4, wobei der Mindestdurchmesser des Stützrings (19) kleiner oder gleich dem Mindestdurchmesser des Anschlagrings (20) ist.

6. Applikator nach einem der Ansprüche 2 bis 5, wobei der Mindestdurchmesser des Stützrings (19) kleiner oder gleich dem Mindestdurchmesser des Positionierungsrings (21) ist.

7. Applikator nach einem der Ansprüche 2 bis 6, wobei der Mindestdurchmesser des Stützrings (19) etwa der gleiche wie der Außendurchmesser der Ausstoßröhre (12) ist.

8. Applikator nach einem der Ansprüche 2 bis 7, wobei der Stützring (19) durch mehrere entlang des Umfangs ausgerichtete Vorsprünge gebildet ist.

9. Applikator nach einem der Ansprüche 2 bis 8, wobei der Anschlagring (20) und/oder der Positionierungsring (21) durch mehrere entlang des Umfangs ausgerichtete Vorsprünge gebildet ist.

10. Applikator nach einem der Ansprüche 2 bis 9, wobei jeweilige gegenüberliegende Neigungen des Anschlagrings (20) und des Positionierungsrings (21) mindestens teilweise eine Vertiefung (22) zwischen diesen bilden.

## Revendications

1. Applicateur de tampon, comprenant :
- un tube éjecteur (12) ;
- un tube externe (3) dimensionné pour s'adapter étroitement et de manière télescopique sur ledit tube éjecteur (12) et ayant une extrémité d'évacuation distale (4) ;
- des moyens de retenue (2) entre lesdits tubes (12 ; 3) pour empêcher le désassemblage dudit tube éjecteur (12) dudit tube externe (3) dans la direction proximale ;
**caractérisé en ce que** lesdits moyens de retenue (2) comprennent :
- des moyens en relief s'étendant de manière circonférentielle (23) ; et
- un ensemble d'au moins trois bagues en relief s'étendant de manière circonférentielle (19 ; 20 ; 21) adjacentes, qui sont respectivement une bague de support (19), une bague de butée (20) et une bague de positionnement (21).

2. Applicateur selon la revendication 1, dans lequel ledit ensemble de bagues (19; 20; 21) est prévu sur la surface proximale interne du tube externe (3) et lesdits moyens en relief (23) sont prévus sur la surface distale externe du tube éjecteur (12).

3. Applicateur selon la revendication 2, dans lequel la bague de support (19) est la bague la plus proche dudit ensemble (19 ; 20 ; 21) de l'extrémité proximale (9) du tube externe (3).

4. Applicateur selon la revendication 3, dans lequel la bague de support (19) est prévue à proximité de l'extrémité proximale (9) du tube externe (3).

5. Applicateur selon l'une quelconque des revendications 2 à 4, dans lequel le diamètre minimum de la bague de support (19) est égal ou inférieur au diamètre minimum de la bague de butée (20).

6. Applicateur selon l'une quelconque des revendications 2 à 5, dans lequel le diamètre minimum de la bague de support (19) est égal ou inférieur au diamètre minimum de la bague de positionnement (21).

7. Applicateur selon l'une quelconque des revendications 2 à 6, dans lequel le diamètre minimum de la bague de support (19) est à peu près le même que le diamètre externe du tube éjecteur (12).

8. Applicateur selon l'une quelconque des revendications 2 à 7, dans lequel la bague de support (19) est formée par une pluralité de saillies alignées de manière circonférentielle.

9. Applicateur selon l'une quelconque des revendications 2 à 8, dans lequel au moins l'une parmi les bagues de butée (20) et de positionnement (21) est formée avec une pluralité de saillies alignées de manière circonférentielle.

10. Applicateur selon l'une quelconque des revendications 2 à 9, dans lequel les inclinaisons se faisant face respectives de la bague de butée (20) et de la bague de positionnement (21) définissent au moins partiellement un creux (22) entre elles.
